# EUROPÄISCHE PATENTSCHRIFT

(11) **EP 1 539 253 B1**
(45) Veröffentlichungstag und Bekanntmachung des Hinweises auf die Patenterteilung: **18.02.2009**
(21) Anmeldenummer: 03770943.3
(22) Anmeldetag: 17.09.2003
(51) Int. Cl.: A61L 27/30, A61L 28/00, A61L 29/10, A61L 31/08

(54) **ANTIINFEKTIÖSE, BIOKOMPATIBLE TITANOXID-BESCHICHTUNGEN FÜR IMPLANTATE SOWIE VERFAHREN ZU DEREN HERSTELLUNG**
ANTI-INFECTIOUS, BIOCOMPATIBLE TITANIUM OXIDE COATINGS FOR IMPLANTS, AND METHOD FOR THE PRODUCTION THEREOF
REVETEMENTS EN OXYDE DE TITANE ANTI-INFECTIEUX BIOCOMPATIBLES POUR IMPLANTS ET LEUR PROCEDE DE REALISATION

(30) Priorität: 17.09.2002 DE 10243132
(43) Veröffentlichungstag der Anmeldung: 15.06.2005
(73) Patentinhaber: Biocer-Entwicklungs-GmbH, 95447 Bayreuth (DE)
(72) Erfinder: ZIEGLER, Günter, 95488 Eckersdorf (DE); GOLLWITZER, Hans, 94469 Deggendorf (DE); HEIDENAU, Frank, 91284 Neuhaus/Pg. (DE); MITTELMEIER, Wolfram, 18069 Rostock (DE); STENZEL, Frauke, 23942 Dassow-Schwanbeck (DE)
(74) Vertreter: Behnisch, Werner
(86) Internationale Anmeldenummer: PCT/EP2003/010334
(87) Internationale Veröffentlichungsnummer: WO 2004/026346

(56) Entgegenhaltungen:
- EP-A2- 0 222 717
- EP-A2- 0 409 810
- US-A- 4 954 476
- US-A- 5 612 049
- US-A- 5 855 612
- US-A- 6 017 553
- US-B1- 6 312 472
- US-B1- 6 313 064
- PATENT ABSTRACTS OF JAPAN vol. 1998, no. 01, 30. Januar 1998 (1998-01-30) -& JP 09 249981 A (NISSHIN STEEL CO LTD), 22. September 1997 (1997-09-22) in der Anmeldung erwähnt

## Beschreibung

Die vorliegende Erfindung betrifft ein Verfahren zur Herstellung einer biokompatiblen metallionenhaltigen Titanoxid-Beschichtung auf einem Implantat, wobei die Metallionen unter physiologischen Bedingungen eluierbar und homogen in der Beschichtung verteilt sind, sowie ein gemäß dem erfindungsgemäßen Verfahren herstellbares Implantat.

### Hintergrund der Erfindung und Stand der Technik

Silber- oder silberhaltige Beschichtungen zur antiinfektiösen Ausstattung von Kurzzeitimplantaten, wie Kathetern, sind klinisch bereits angewendete Verfahren, und die antibakterielle Wirkung ist aus der Literatur bekannt [1-3]. Die antibakterielle Wirkung von Kupfer bzw. Kupferionen wurde bis jetzt hauptsächlich anhand von metallischen Filmen, also an reinen Kupferoberflächen untersucht [4]. Eine Verwendung von elementarem Kupfer als Beimengung wird für eine antibakterielle Wandfarbe beschrieben [5]. Die Elution von Kupferionen aus Kupfer-Thiomolybdatkomplexen in das Blut von Ratten wird von Komatsu, et al. beschrieben [6]. Kupferhaltige Titanoxidschichten, die durch thermische Oxidation einer kupferhaltigen Titanlegierung erhalten werden, werden in den japanischen Offenlegungsschriften JP9118987 und JP9249981 offenbart. Bei diesen Verfahren handelt es sich aber nicht um eigentliche Beschichtungsverfahren bzw. Beschichtungen, sondern es wird lediglich die Oberfläche von kupferhaltigen Titanlegierungen durch Säurebehandlung verändert. Die französische Patentanmeldung FR2780417 beschreibt ein ähnliches Verfahren, wobei jedoch die Oberfläche der behandelten Legierung vor der Oxidation zu einer Oberflächenoxidschicht mit einer oxidierenden Mineralsäure behandelt wird. Die Herstellung von biokompatiblen Titanoxid-Beschichtungen aus Nanosuspensionen, sog. Solen, ist beispielsweise aus [7] und [8] bekannt.

In klinischen Umgebungen sind bakterielle Verunreinigungen eine latente und nicht zu vermeidende Gefahr, insbesondere im chirurgischen Bereich, z.B. bei operativen Eingriffen am Patienten. Vor allem bei der Einbringung von Fremdkörpern (Implantate wie Katheter, Osteosyntheseplatten, Endoprothesen usw.) findet direkt nach der Implantation ein in der Literatur als "race for the surface" beschriebener Vorgang statt [11]. Dabei kommt es zu einem Wettlauf zwischen den körpereigenen Zellen und den während der Operation mit eingebrachten Mikroorganismen um die Besiedelung der zunächst sterilen Implantatoberflächen. Kommt es zu einer übermäßigen Erstbelegung mit Bakterienzellen auf der Implantatoberfläche und Ausbildung einer manifesten Infektion, werden dadurch die Immunmechanismen des menschlichen Körpers in Gang gesetzt, und das Implantat wird möglicherweise abgestoßen. Von Bakterien besiedelte Implantate müssen in den meisten Fällen zur Therapie der Infektion entfernt werden, da selbst hohe Konzentrationen wirksamer Antibiotika keine komplette Eradikation adherierender Bakterien erreichen [12, 13]. Werden die Implantatoberflächen jedoch stark toxisch gestaltet, so wird gleichzeitig auch eine Belegung mit körpereigenen Zellen, die für die Integration des Implantats notwendig ist, verhindert. Dies ist insbesondere bei Langzeitimplantaten, wie Hüftendoprothesen, ein unerwünschter Effekt. Eine Besiedelung mit vitalen Körperzellen fördert die Integration des Implantates und erschwert eine Infektion.

Es ist daher eine Aufgabe der vorliegenden Erfindung, eine Beschichtung für Implantate bereitzustellen, welche das Wachstum eingeschleppter Mikroorganismen auf diesen Implantaten, insbesondere das Wachstum von Bakterien, verhindert und im Anschluss daran für körpereigene Zellen eine biokompatible Oberfläche zur Verfügung stellt.

Dies wird erfindungsgemäß durch eine Verfahren gemäß Anspruch 1 und ein gemäß diesem Verfahren herstellbares Implantat gelöst. Beschrieben ist auch die Verwendung des Implantats für die Implantation in Patienten. Weitere Ausgestaltungen der Erfindung ergeben sich aus den Unteransprüchen und der nachfolgenden Beschreibung.

### Beschreibung

Erfindungsgemäß wird ein Verfahren zur Herstellung einer Metallionen enthaltenden, biokompatiblen Titanoxid-Beschichtung auf einem Implantat bereitgestellt, mit dem ein Implantat herstellbar ist, bei dem die Metallionen unter physiologischen Bedingungen aus der Beschichtung in die Umgebung abgegeben werden und wobei die Metallionen homogen in der Beschichtung verteilt sind.

Unter Implantat ist erfindungsgemäß ein Substrat zu verstehen, dass dafür geeignet ist, in einen Patienten implantiert zu werden. Beispiele für Implantate sind Katheter, Osteosynthesematerial, Endoprothesen, Fixateurs externes/internes, Nägel, Schrauben und/oder Drähte, Herzklappen, künstliche Blutgefäße und Shunts, gesichtschirurgische/plastische Implantate, Mittelohrimplantate, Dentalimplantate, etc.

Nach dem Stand der Technik werden Titanoxid-Beschichtungen durch Oxidation von Titan bei erhöhten Temperaturen oder beispielsweise durch Plasmaspritzmethoden hergestellt. Hierbei ist es nicht möglich, eine homogene Einbringung von Metallionen zu erreichen. Beispielsweise werden mit bekannten physikalischen Beschichtungsverfahren wie PVD (Physical Vapour Deposition) keine gleichmäßigen, sondern stets inselförmige Abscheidungen auf Oberflächen erzeugt, wodurch sich das Risiko einer lokalen Toxizität ergibt. Bei dem aus dem Stand der Technik bekannten Verfahren zur Einbringung von Metallen oder Metallionen in eine Matrix wurden diese stets in Form mikrometergroßer Pulver oder ebenso dimensionierter salzartiger Verbindungen in pulvermetallurgischer Art und Weise zu Pulvern zugemischt und trocken verpresst, wobei eine nur inhomogene Verteilung des Materials in der Suspension und damit auch im fertigen Material erreicht wird [9, 10].

Die vorliegende Erfindung betrifft dagegen eine Titanoxid-Beschichtung bzw. ein mit einer Titanoxid-Beschichtung versehenes Implantat, wobei in der Beschichtung Metallionen enthalten sind, die homogen in der Beschichtung verteilt sind und unter physiologischen Bedingungen eluierbar sind. Die Metallionen liegen dabei in einer solchen Konzentration in der Beschichtung vor, dass diese zunächst unter physiologischen Bedingungen eine antimikrobielle bzw. antibakterielle Wirkung durch die darin enthaltenen Metallionen entfalten kann, ohne dabei körpereigene Zellen wesentlich zu schädigen. Bei der zunächst vorliegenden Konzentration dieser Ionen in der Beschichtung werden diese unter physiologischen Bedingungen herausgelöst, so dass sie an der Beschichtungsoberfläche ihre antimikrobielle Wirkung entfalten können.

Unter physiologischen und pathophysiologischen Bedingungen sind erfindungsgemäß Bedingungen zu verstehen, die in der Umgebung eines in einen Patienten implantierten Implantats vorherrschen können. Der Begriff umfasst erfindungsgemäß sämtliche Körperflüssigkeiten, die mit einem solchen implantierten Implantat in Kontakt kommen, aber auch sonstige Pufferlösungen, welche als Ersatz für Körperflüssigkeiten eingesetzt werden, wie z.B. eine physiologische Kochsalzlösung, Phosphat-gepuffertes Kochsalz (PBS) und ähnliches.

Nach einiger Zeit sinkt die Konzentration in der Beschichtung so weit ab, dass die antimikrobielle bzw. antibakterielle Wirkung nicht mehr auftritt und die verbleibende Schicht nunmehr für körpereigene Zellen uneingeschränkt kompatibel ist. Die antibakterielle Wirkung kann dabei zusätzlich durch die Steuerung der Schichtzusammensetzung genau dosiert werden. So kann es beispielsweise sinnvoll sein, Implantate für besonders infektionsgefährdete Implantatlager mit einer höheren Metallionenkonzentration zu versehen (z. B. Marknägel im Rahmen von offenen Knochenbrüchen, Fixateur externe mit Steinmann-Nägel oder Pins bei Osteomyelitis, temporäre Spacer bei infizierten Endoprothesen im Rahmen von sog. zweizeitigen Wechseleingriffen). Die Konzentration an Metallionen darf eine toxische Konzentration jedoch nicht überschreiten, da sonst eine Schädigung des Wirtsorganismus erfolgen würde. Andererseits sollte die Grenzkonzentration der antibakteriellen Wirkung nicht unterschritten werden, bis die bei der Implantation eingeschleppten Bakterien abgestorben sind.

Allgemein können die Konzentrationen an Metallionen in der Titanoxid-Beschichtung bevorzugt 0,1 - 20 Gew.-%, bezogen auf die Gesamtbeschichtung, betragen, bevorzugt 5 - 15 Gew.%, noch bevorzugter 10 - 12 Gew.-%.

Unter Titanoxid ist erfindungsgemäß im wesentlichen Titandioxid zu verstehen. Es ist jedoch auch Titanoxid mit anderen Wertigkeiten des Titan erfindungsgemäß umfasst, sowie Mischungen hiervon mit Titandioxid, solange diese Titanoxide keine nachteilige Wirkung hinsichtlich der Biokompabilität und Toxizität aufweisen.

Die Dicke der erfindungsgemäßen Titanoxid-Beschichtung liegt im Bereich von einigen Hundert Nanometern, bevorzugt ca. 50 bis 1000 nm, bevorzugter 50 - 200 nm, noch bevorzugter 130 - 170 nm, am bevorzugtesten etwa 150 nm.

Als Implantate können erfindungsgemäß metallische Implantate, Implantate aus Metalllegierungen, Kunststoffe, Gläser, keramische Implantate, Verbundwerkstoffe oder Kombinationen aus diesen verwendet werden. Beispiele für bevorzugte Implantate sind Katheter, Osteosyntheseplatten, Endoprothesen, Fixateurs externes/internes, Nägel, Schrauben und/oder Drähte, Herzklappen, künstliche Blutgefäße und Shunts, gesichtschirurgische/plastische Implantate, Mittelohrimplantate und Dentalimplantate.

Beispiele für Metalle und Metalllegierungen, die erfindungsgemäß bevorzugt eingesetzt werden können, sind Titan, Stahl, Eisen und/oder Stahl-, Eisen-, Titanlegierungen, Kobalt-Chrom-Basislegierungen und/oder Osteosynthesestahl, bevorzugt AISI316L). Besonders bevorzugt sind Titanlegierungen. Unter den Titanlegierungen sind TiAl6V4 und Ti-A16Nb7 besonders bevorzugt.

Beispiele für Kunstoffe, die erfingdungsgemäß bevorzugt eingesetzt werden können, sind Polymere wie Polyethylen, Polypropylen, Polytetrafluorethylen, Polyethylenterephthalat, Polyamide, Polyurethane, Polysiloxane, Polysiloxan-Elastomere, Polyetheretherketon und Polysulfon.

Beispiele für keramische Materialien, die erfindungsgemäß bevorzugt eingesetzt werden können, sind Aluminiumoxid, Zirkonoxid, Hydroxylapatit, Gläser und Glaskeramiken.

Es ist erfindungsgemäß notwendig, dass die Metallionen in der Titanoxid-Beschichtung homogen verteilt sind, da menschliche Zellen und Bakterien sehr empfindlich auf Konzentrationsgradienten reagieren und deshalb bei einer lokalen Verteilung bzw. Konzentrierung im Mikrometermaßstab eine gleichmäßige Wirkung über die gesamte Fläche der Beschichtung nicht garantiert ist. Unter homogen ist erfindungsgemäß also zu verstehen, dass die Metallionen im wesentlichen molekular bzw. atomar verteilt vorliegen und im wesentlichen keine Aggregate bilden, die einen Durchmesser von wenigen Nanometern übersteigen.

Eine solche homogene Verteilung kann erfindungsgemäß erreicht werden, indem zur Herstellung der Titanoxid-Beschichtung auf dem Implantat eine Beschichtungszubereitung bzw. -suspension hergestellt und zum Aufbringen auf das Implantat verwendet wird, in der Metallionen gelöst sind.

Das erfindungsgemäße Verfahren zur Beschichtung von Substraten bzw. Implantaten weist die folgenden Schritte auf. Zunächst wird eine Zubereitung, die als eine dünnflüssige Suspension, ein sogenanntes Sol, enthaltend ein organisches Lösungsmittel, einen metallorganischen Titanoxid-Precursor sowie wahlweise Wasser und/oder eine Säure, bevorzugt eine mineralische Peptisiersäure, hergestellt und mit Metallverbindungen (Metallsalze und/oder metallorganische Verbindungen) versetzt wird. Unter Sol ist erfindungsgemäß eine kolloidale Lösung zu verstehen, in der ein fester oder flüssiger Stoff in feinster, d.h. im wesentlichen in molekularer bzw. atomarer Verteilung ohne Aggregatbildung in einem flüssigen Medium dispergiert ist. Erfindungsgemäß sind die Metallsalze und/oder Metallverbindungen in dem Sol bevorzugt vollständig gelöst. Das Sol kann auch als Nanosuspension bezeichnet werden, da die Metallverbindungen oder -ionen im Nanometerbereich verteilt vorliegen.

Anschließend erfolgt das Aufbringen der so hergestellten Zubereitung auf ein Implantat und ein Trocknen der aufgebrachten Beschichtung. Wahlweise kann sich ein Trocknungsschritt bei 100 - 1000°C anschließen.

Durch das erfindungsgemäße Verfahren konnte eine Titanoxid-Beschichtung bzw. ein Implantat mit einer Titanoxid-Beschichtung bereitgestellt werden, wobei die Metallionen mit antimikrobieller Wirkung unter physiologischen Bedingungen herauslösbar sind, wobei eine antimikrobielle Wirkung insbesondere im nahen Bereich der Titanoxid-Beschichtung erzielt werden kann. Nach einer gewissen Zeit, wenn die antimikrobiell wirkenden Metallionen im wesentlichen herausgelöst sind, nimmt die antimikrobielle Wirkung der Beschichtung ab und das Implantat wird vom Körpergewebe integriert, ist also biokompatibel. Die erfindungsgemäßen Implantate sind damit besonders zur Implantation in Patienten geeignet. Bekannte kupferhaltige Materialien behalten dagegen ihre antimikrobielle Wirkung über den gesamten Einsatzzeitraum, was zu einer chronisch entzündlichen Reaktion mit fehlender Integration führt. Die vorliegende Erfindung ermöglicht eine definierte Abgabe von z.B. Kupfer über einen einstellbaren Zeitraum, um die Proliferation von am Implantat haftenden Bakterien zu stoppen, ohne die anhaftenden körpereigenen Zellen übermäßig zu schädigen. Danach stellt die Beschichtung aber ein biokompatibles Material dar, das ein Anwachsen von körpereigenem Gewebe an das Implantat zulässt.

Metallverbindungen sind bevorzugt lösliche Salze oder metallorganische Verbindungen oder Komplexe daraus. Diese werden in definierter Menge in die dünnflüssige Suspension bzw. das Sol eingebracht und gelöst.

Anschließend wird dieses nahezu wasserflüssige Gemisch auf das Substrat aufgebracht. Dies kann durch Tauchbeschichtung, Spin-Coaten, Rakeln, Drucken oder Aufsprühen oder andere Verfahren gemäß dem Stand der Technik geschehen.

Die in dem Gemisch enthaltenen Titanoxid-Precursor sind vierfach koordinierte Titanverbindungen mit sauerstoffverbrückten linearen oder verzweigten Alkylresten mit einer bevorzugten Kettenlänge von C2 bis C5. An Stelle oder zusätzlich können ungesättigte Alkylreste (Alkenylreste) und/oder Sauerstoff- und/oder Stickstoff-haltige Alkylreste bzw. Alkenylreste erfindungsgemäß für spezielle Anwendungen wie UV-Härtbarkeit ebenfalls verwendet werden, die ebenfalls bevorzugt 2 bis 5 C-Atome, aber auch längere Alkylketten bis C-12, aufweisen. Beispiele für geeignete sauerstoffverbrückte Alkylreste sind insbesondere Ethyl-, n-Propyl-, Isopropyl-, n-Butyl-, Isobutyl-, n-Pentyl- und/oder Isopentylreste.

Beispiele für geeignete Alkenylreste sind Acrylate, Methacrylate oder längerkettige Alkylketten oder verzweigte Alkylketten, die Doppelbindungen tragen. Die bevorzugte Kettenlänge der Hauptkette beträgt C2 bis C12, die der Seitenketten C2 bis C6.

Beispiele für geeignete 0-substituierte und N-substituierte Alkyl- und/oder Alkenylreste sind kohlenstoffkettenbasierte Reste, die den bereits beschriebenen Anforderungen genügen, zusätzlich jedoch Ether-, Keto- oder Aminogruppen enthalten.

Beispiele für erfindungsgemäß verwendbare Titanoxid-Precursor sind Tetrabutoxytitanat, Titanisobutoxytitanat, Titantetrapropylat, Titantetraisopropylat, Titantetraacetylacetonat, Titantetraethoxytitanat.

Als organisches Lösungsmittel werden bevorzugt lineare oder verzweigte Alkohole mit Kettenlängen von 2 bis 8 Kohlenstoffatomen verwendet, z.B. Ethanol, Propanol, Isopropylalkohol, n-Butanol, sec-Butanol oder Kombinationen aus den genannten Alkoholen, wobei Ethanol und n-Butanol besonders bevorzugt sind. Weitere organische Lösungsmittel, die erfindungsgemäß einsetzbar sind, sind cyclische, aromatische und/oder heteroaromatische Kohlenwasserstoffe oder deren Derivate, bspw. Cyclopentan, Cyclohexan, Benzol, Toluol, Tetrahydrofuran oder Dioxan, wobei Benzol, Toluol und/oder Tetrahydrofuran besonders bevorzugt sind. Das organische Lösungsmittel kann vom Fachmann entsprechend dem verwendeten Metallsalz oder der metallorganischen Verbindung gewählt werden.

Wahlweise kann in der Zubereitung Wasser und/oder eine Säure, vorzugsweise eine mineralische Peptisiersäure, enthalten sein.
Als mineralische Peptisiersäure wird bevorzugt Salpetersäure verwendet. Es können jedoch zusätzlich oder stattdessen andere Peptisiersäuren, wie Salzsäure, Schwefelsäure, Phosphorsäure, oder organische Säuren wie Zitronensäure oder Essigsäure eingesetzt werden.

Bei Verwendung einer Säure bzw. Peptisiersäure beträgt die Konzentration der Säure bzw. Peptisiersäure bevorzugt 1 bis 50 mol% des eingesetzten Titanoxid-Precursors, bevorzugter 2 bis 20 mol%, noch bevorzugter 8 bis 10 mol%.

Die Konzentration des Lösungsmittels beträgt bevorzugt das 5 bis 50-fache der Molmenge des Titanoxid-Precursors, bevorzugter das 15 bis 40-fache, noch bevorzugter das 20 bis 35-fache.

Der Anteil der Metallverbindungen entspricht bevorzugt einer Kaltsättigung der Beschichtungslösung. Entsprechende Verdünnungen sind stufenlos möglich und werden dem Anwendungsfall angepasst. Ebenfalls bevorzugt wird die Metallionen-Konzentration in der Beschichtungs so gewählt, dass die aufgebrachte getrocknete und ggf. erhitzte Titanoxid-Beschichtung eine Metallionenkonzentration von 1 - 20 Gew.-%, bevorzugt 5 - 15 Gew.%, noch bevorzugter 10 - 12 Gew.-% aufweist.

Die in der Beschichtungszubereitung verwendeten Metallsalze und/oder metallorganischen Verbindungen weisen ein- bis vierwertige Metallionen auf, ausgewählt aus Zink-, Queck-silber-, Kupfer- und Silbersalzen. Als Gegenionen können Nitrate, Sulfate, Carbonate, Hydroxide, bevorzugt aber Acetate und Chloride eingesetzt werden. Erfindungsgemäße Beispiele sind z.B. Kupferacetat, Kupferchlorid, Silberacetat.

Bei oder nach dem Aufbringen der oben beschriebenen Zubereitung, vorzugsweise in Form eines Sols, auf das Substrat kann erfindungsgemäß erreicht werden, dass dieses durch Abdampfen des Lösungsmittels und/oder durch Einstellung von stöchiometrischen Eduktverhältnissen in ein verfestigtes formbeständiges aber ein leicht deformierbares System bzw. ein Gel übergeht, wobei die Metallionen homogen echt gelöst in dem verfestigten System oder Gel vorliegen und damit im wesentlichen molekular dispergiert sind. Sol-Gel-Verfahren zur Beschichtung von Materialien sind an sich bekannt, nicht jedoch die erfindungsgemäße Abwandlung hiervon zur Einbringung von eluierbaren Metallionen in die Beschichtung.

Anschließend erfolgt eine Trocknung, wonach die beschichteten Implantate direkt verwendet werden können. Wahlweise erfolgt auch eine Wärmebehandlung bei Temperaturen von 100 bis 1000°C, für ungefähr 0,1 - 3 Stunden, bevorzugt 0,1 - 1 Stunden die unter Sauerstoff-, Stickstoff-, Argon- oder Luftatmosphäre erfolgen kann. Diese nachfolgende Wärmebehandlung erfolgt zur mechanischen Stabilisierung bzw. zur Verdichtung der Beschichtungen. Z.B. kann durch das Erhitzen bei etwa 500°C bevorzugt eine Keramisierung der Beschichtung erfolgen. Bei Kunststoffimplantaten erfolgt bevorzugt ein weniger starkes Erhitzen.

Wahlweise erfolgt der Trocknungsschritt unter überkritischen Bedingungen, bevorzugt in einem Autoklaven. Unter "überkritischen Bedingungen", wie sie hierin genannt sind, ist bei festgelegtem Autoklavenvolumen ein Druck-Temperatur-Zeit Profil zu verstehen, bei welchem das verwendete Lösungsmittel ohne Ausbildung einer Phasengrenze durch Dichteerniedrigung vom flüssigen in den gasförmigen Zustand jenseits des physikalisch definierten kritischen Punktes gebracht wird und so aus der Schicht entfernt wird.

Die besonderen Vorteile dieses Verfahrens liegen in der Beibehaltung der geltypischen, nanometerskaligen Porenstruktur und damit der Schaffung einer sehr hohen spezifischen Oberfläche der Beschichtung. Dadurch ist es einerseits möglich, die Ionenfreisetzungskinetik der Kupferionen zusätzlich zu beeinflussen und andererseits durch die Schaffung einer strukturierten, porösen Oberfläche das Wachstum von Körperzellen wie z.B. Osteoblasten oder Fibroblasten positiv zu beeinflussen.

Wahlweise erfolgt auch eine Wärmebehandlung bei Temperaturen von 100 bis 1000°C, für ungefähr 0,1 - 3 Stunden, bevorzugt 0,1 - 1 Stunden die unter Sauerstoff-, Stickstoff-Argon- oder Luftatmosphäre erfolgen kann. Diese nachfolgende Wärmebehandlung erfolgt zur mechanischen Stabilisierung der Beschichtungen. Z.B. kann durch das Erhitzen bei etwa 500°C bevorzugt eine Keramisierung der Beschichtung erfolgen. Bei Kunststoffimplantaten erfolgt bevorzugt ein weniger starkes Erhitzen.

Durch das erfindungsgemäße Verfahren ist es möglich, durch Verdünnung bzw. Mehrfachbeschichtung, die Konzentration an Metallionen, bevorzugt Kupfer- und/oder Silberionen, in der Beschichtung genau einzustellen. Durch Mehrfachbeschichtung kann die antimikrobielle Wirkung der Beschichtung verstärkt werden, da dann eine größere Menge antibakteriell wirkender eluierbarer Metallionen bereitgestellt werden kann. Bevorzugt ist eine Zweifach- bis Vierfachbeschichtung.

Eine Mehrfachbeschichtung wird erfindungsgemäß hergestellt, indem die Schritte zur Herstellung einer Titanoxid-Beschichtung auf einem Implantat, d.h. Versetzen einer Zubereitung, enthaltend ein organisches Lösungsmittel und einen metallorganischen Titanoxid-Precursor und wahlweise Wasser und/oder eine Säure, mit Metallsalzen und/oder mit metallorganischen Verbindungen, um Metallionen homogen in der Zubereitung zu verteilen, Aufbringen der hergestellten Zubereitung auf ein Implantat und Trocknen der aufgebrachten Beschichtung, ein oder mehrmals wiederholt werden, so dass eine oder mehrere zusätzliche Titanoxid-Beschichtungen auf dem Implantat erzeugt werden. Wahlweise kann jeweils nach der Durchführung der vorgenannten Verfahrensschritte ein Erhitzen auf 100 bis 1000°C durchgeführt werden.

Vorzugsweise werden die Metallionen-Konzentrationen jeweils so variiert, dass die ein oder mehreren zusätzlich aufgebrachten getrockneten und ggf. erhitzten Beschichtungen unterschiedliche Metallionenkonzentrationen bzw. auch unterschiedliche Metallionen aufweisen, wobei die Metallionen-Konzentrationen jeweils besonders bevorzugt so variiert werden, dass die Metallionenkonzentration in den Beschichtungen von den innen am Implantat liegenden Beschichtungen hin zu den außen liegenden Beschichtungen abnehmen.

Die Vorteile dieser Vorgehensweise liegen in der genauen Einstellbarkeit der Freisetzungskinetik und damit verbunden der einsatzortspezifischen Ausbildung der Beschichtung. So ist es beispielsweise möglich, direkt nach der Implantation durch Einbringen des stark bakterizid wirkenden Silbers in die äußerste Schicht eine schnelle Keimzahlreduktion zu erhalten. Durch eine spätere Elution von Kupfer aus innenliegenden Schichten wird anschließend die Keimzahl niedrig gehalten, ohne das Wachstum von an der Integration des Implantats im Körper beteiligten Zellen zu behindern.

Auch bei einer Mehrfachbeschichtung sind die Metallionen in den einzelnen Beschichtungen jeweils homogen verteilt.

Im Folgenden werden einige Beispiele beschrieben, die jedoch den Umfang der Erfindung nicht einschränken sollen.

In den Beispielen wird auf folgende Figuren Bezug genommen:
Fig. 1 zeigt die Zellzahlentwicklung von S. aureus ATCC 25923 nach 24h Kultur auf unterschiedlichen Materialoberflächen.
Fig. 2 zeigt die Zellzahlentwicklung von Mausfibroblasten (L929) nach 24h Kultur auf unterschiedlichen Materialoberflächen.

### Beispiele

### Beispiel 1: Beschichtung

69,5 g Tetrabutoxytitanat werden in 500 g n-Butanol bei RT gelöst und unter Inertgasbedingungen 2 h gerührt. Dann wird bis zur Kaltsättigung portionsweise Kupferacetat zugegeben. Der Überstand wird vom Bodensatz abgezogen und als Beschichtungslösung verwendet.

Die Beschichtung erfolgt durch Tauchen des Probekörpers TiAl6V4, Glas oder Kunststoff mit einer Abzugsgeschwindigkeit von 1,5 mm/s. Anschließend wird bei Raumtemperatur 1h getrocknet und die Beschichtung bei 500 °C 10 min keramisiert.

Bei der Beschichtung von Kunststoff fällt der Keramisierungsschritt weg, dafür wird nach dem Trocknen 1 h bei 120 °C getempert.

### Beispiel 2: Darstellung der Wirkungsweise

Um die Wirkungsweise der antibakteriellen Beschichtung zu demonstrieren wurden einerseits Untersuchungen an klinisch relevanten Bakterienstämmen (Staphylococcus aureus: ATCC25923, MRSA27065 und Staphylococcus epidermidis: ATCC35984, RP62a, SE 183) und andererseits an Bindegewebszellen (L929, Mausfibroblasten) und foetalen Osteoblasten (MC3T3-E1) durchgeführt. Als Probenmaterial dienten erfindungsgemäß antibakteriell beschichtete TiA16V4 Plättchen (Durchmesser 14,5 mm, Dicke 1 mm). Zur direkten Vergleichbarkeit wurden die Versuche mit Zellen und Bakterien im gleichen Zellkulturmedium unter gleichen Bedingungen durchgeführt (Kulturmedium: 90% RPMI 1640 (= 2,05mM glutaminhaltiges Serum), 10% FKS (fötales Kälberserum); Inkubation: 24h, 37°C, 5% CO2, statische Kultur, Dunkelheit).
- *Zell-Linien:*: *MC3T3-E1 (Mausosteoblasten),*
*L929 (Mausfibroblasten)*
24-Well Kulturschalen, Polystyrol
Inokulum: 120.000 Zellen/ml und Well (Vertiefung), lg-Phase, Passage 6

Zellproliferation: Trypsinierung (300 µl Trypsin-EDTA) für die Dauer von 8 Minuten im Schüttelinkubator bei 37°C, Abstoppen der Enzymreaktion mit 700 µl Kulturmedium.
Bestimmung der Zellzahl im Coulter-Counter.
*Bakterienstämme (ATCC25923, MRSA27065, ATCC35984, RP62a, SE 183)*
Sämtliche Versuche wurden korrespondierend zu den Zelltests durchgeführt
Inokulum: 100.000 Zellen/mL und Well

Das Ablösen adherierender Mikroorganismen erfolgte mittels Ultraschall, die Bakterienzahl wurde quantitativ nach Verdünnung durch Auszählung der "Kolonie bildenden Einheiten (KBE)" nach 24 Stunden bei 37°C auf Nährböden (Müller-Hinton-Agarplatten) ermittelt und die unverdünnte Keimzahl berechnet. Dabei werden nur die vitalen Bakterien gezählt, da abgetötete oder inaktive Keime keine KBEs bilden.

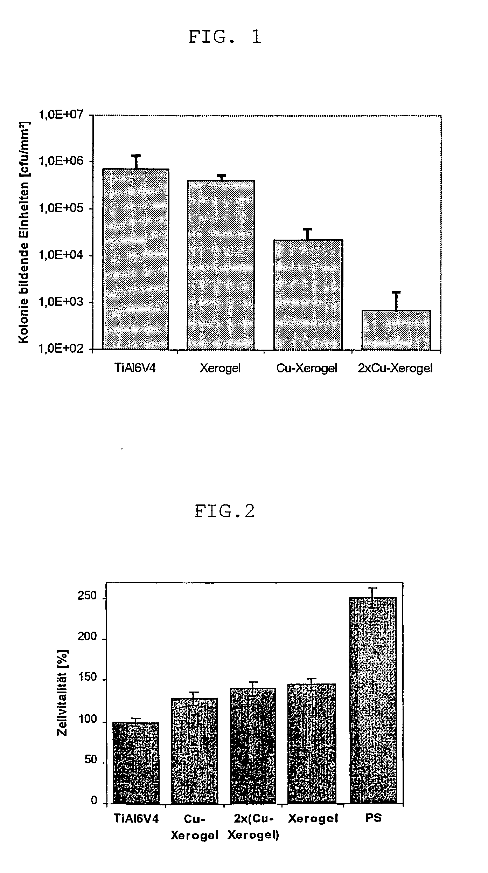

Fig.1 zeigt die Zellzahlentwicklung von S. aureus ATCC 25923 nach 24h Kultur auf unterschiedlichen Materialoberflächen.
- TiAl6V4:: Referenz, reine Legierung
- Cu-Xerogel:: TiAL6V4, das einfach mit einer erfindungsgemäßen kupferhaltigen Titanoxidbeschichtung wie in Bsp. 1 versehen wurde
- 2x Cu-Xerogel:: TiAL6V4, das mit einer doppelten erfindungsgemäßen kupferhaltigen Titanoxidbeschichtung versehen wurde
- Xerogel:: Reine Titanoxidbeschichtung ohne Zugabe von Kupfer

Fig. 2 zeigt die Zellzahlentwicklung von osteoblastenähnlichen Zellen (MC3T3-E1) nach 24h Kultur auf unterschiedlichen Materialoberflächen:
- TiAl6V4:: Referenz, reine Legierung
- Cu-Xerogel:: TiAL6V4, das einfach mit einer erfindungsgemäßen kupferhaltigen Titanoxidbeschichtung wie in Bsp. 1 versehen wurde
- 2x Cu-Xerogel:: TiAL6V4, das mit einer doppelten erfindungsgemäßen kupferhaltigen Titanoxidbeschichtung versehen wurde
- Xerogel:: Reine Titanoxidbeschichtung ohne Zugabe von Kupfer
- PS:: Polystyrol als Kontrolle

Es ist aus Figur 2 ersichtlich, dass bei einer einfachen erfindungsgemäßen Kupferbeschichtung die Zellzahl der Fibro-blasten im Vergleich zur Metalllegierung zunimmt. Bei einer doppelten kupferhaltigen Schicht (entsprechend doppelter Menge Kupfer im System) liegen die Zellzahlen im Rahmen des Fehlers sogar auf gleichem Niveau mit der einfach xerogel-beschichteten Legierung.

Bei den Bakterienstämmen (Fig. 1) ist deutlich zu erkennen, dass bereits bei einer erfindungsgemäßen Einfachbeschichtung die Zellzahl um zwei Zehnerpotenzen abnimmt.

Bei einer Doppelbeschichtung erniedrigt sich die Zellzahl noch deutlicher.

Eine ebenfalls erfindungsgemäße Vierfachbeschichtung ergibt eine Zellzahlreduktion um 6 Zehnerpotenzen, was mikrobiologisch einer Sterilisation gleichzusetzen ist.

Eine vergleichbare Reduktion des bakteriellen Wachstums wurde in der Inkubationslösung erreicht, welche die beschichteten Metallproben umgab. Dadurch wird deutlich, dass tatsächlich eine Elution von Kupferionen in das Kulturmedium stattfindet und die antibakterielle Wirkung kein reiner Oberflächeneffekt ist.

### Literatur

[1] Illingworth B., Bianco R. W., Weisberg S., In vivo efficacy of silver-coated fabric against Staphylococcus epidermidis, J Heart Valve Dis 1 (2000) 135-41.
[2] Darouiche R. O., Anti-infective efficacy of silver-coated medical prostheses, Clin Infect Dis 6 (1999) 1371-7.
[3] Ambrosius W. T., Harris V. J., Snidow J. J., Tunneled hemodialysis catheters: use of a silver-coated catheter for prevention of infection - a randomized study, Radiology 2 (1998) 491-6.
[4] Grzybowski J., Trafny E. A., Antimicrobial properties of copper-coated electro-conductive polyester fibers, Polim Med 29 (1999) 27-33.
[5] Cooney T. E., Bactericidal activity of copper and noncopper paints, Infect Control Hosp Epidemiol 16 (1995) 444-50.
[6] Komatsu Y., Sadakata I., Ogra Y., Suzuki K. T., Excretion of copper complexed with thiomolybdate into the bile and blood of LEEC rats, Chem Biol Interact 124 (2000) 217-31.
[7] Jokinen M., Pätsi M., Rahiala H., Peltola T., Ritala M., Rosenholm J. B., Influence of sol and surface properties on in vitro bioactivity of sol-gel-derived TiO2 and TiO2-SiO2 films deposited by dip-coating method, J. Biomed. Mater. Res. 42 (1998) 295-302.
[8] Heidenau F., Schmidt H., Stenzel F., Ziegler G., Sol-Gel-Derived Titania with Gradient Porosity, Key. Eng. Mater. 161-163 (1999) 115-116.
[9] Feng Q.L., Cui F.Z., Kim T.N., Kim J.W., Agsubstituted hydroxyapatite coatings with both antimicrobial effects and biocompatibility, J Mater Sci Lett 18 (1999) 559-61.
[10] Shirkhanzadeh M., Azadegan, M., Formation of carbonate apatite on calcium phosphate coatings containing silver ions, J Mater Sci: Mater Med 9 (1998) 385-91.
[11] Gristina A.G., Biomaterial-centered infection: Microbial adhesion versus tissue integration, Science 237 (1987) 1588-95.
[12] Dunne W.M.Jr., Mason E.O.Jr., Kaplan S.L., Diffusion of rifampin and vancomycin through a Staphylococcus epidermidis biofilm. Antimicrob. Agents Chemother.. 37 (1993) 2522-6.
[13] Darouiche R.O., Dhir A., Miller A.J., Landon G.C., Raad I.I., Musher D.M., Vancomycin penetration into biofilm covering infected prostheses and effect on bacteria, J. Infect. Dis. 170 (1994) 720-3.

## Patentansprüche

1. Verfahren zur Herstellung einer Titanoxid-Beschichtung auf einem Implantat mit den Schritten:
a) Versetzen eines Sols, enthaltend ein organisches Lösungsmittel und einen metallorganischen Titanoxid-Precursor und wahlweise Wasser und/oder eine Säure, mit Metallsalzen und/oder mit metallorganischen Verbindungen, um Metallionen homogen in dem Sol zu verteilen, wobei die Metallionen unter physiologischen Bedingungen eine antimikrobielle bzw. antibakterielle Wirkung entfalten und ein- bis vierwertige Metallionen aufweisen, ausgewählt aus Kupfer-, Zink-, Silber- und Quecksilberionen, und wobei der metallorganische Titanoxid-Precursor vierfach koordiniertes Titan mit sauerstoffverbrückten, linearen oder verzweigten Alkyl- und/oder Alkylenresten, die in der Kette oder substituiert O- und/oder N-Atome aufweisen können, ist,
b) Aufbringen des unter a) hergestellten Sols auf ein Implantat,
c) Trocknen der aufgebrachten Beschichtung.

2. Verfahren nach Anspruch 1,
**dadurch gekennzeichnet,**
**dass** nach Schritt c) ein Erhitzen auf 100 bis 1000°C durchgeführt wird.

3. Verfahren nach Anspruch 1 oder 2,
**dadurch gekennzeichnet,**
**dass** das Implantat ein Metall, eine Metalllegierung, ein Glas, eine Keramik, ein Kunststoff, ein Verbundwerkstoff oder ein Knochenimplantat ist.

4. Verfahren nach einem oder mehreren der vorhergehenden Ansprüche,
**dadurch gekennzeichnet,**
**dass** das Implantat ein Katheter, eine Osteosyntheseplatte, eine Endoprothese, ein Fixateur externe, ein Fixateur interne, ein Nagel, eine Schraube oder ein Draht, eine Herzklappe, ein künstliches Blutgefäß oder ein Shunt, ein gesichtschirurgisches/plastisches Implantat, ein Mittelohrimplantat oder ein Dentalimplantat ist.

5. Verfahren nach einem oder mehreren der vorhergehenden Ansprüche,
**dadurch gekennzeichnet,**
**dass** das Metall im Fall eines Metallimplantants Titan, Stahl, Eisen ist und/oder eine Stahl-, Eisen-, Titan- und/oder CoCr-Legierung.

6. Verfahren nach einem oder mehreren der vorhergehenden Ansprüche,
**dadurch gekennzeichnet,**
**dass** die Metalllegierung eine Titanlegierung, bevorzugt TiA16V4 oder TiA16Nb7, eine CoCr-Legierung oder ein Osteosynthesestahl ist.

7. Verfahren nach einem oder mehreren der vorhergehenden Ansprüche,
**dadurch gekennzeichnet,**
**dass** der Kunststoff Polyethylen, Polypropylen, Polytetrafluorethylen, Polyethylenterephthalat, Polyamide, Polyurethane, Polysiloxane, Polysiloxan-Elastomere, Polyetheretherketon und/oder Polysulfon ist.

8. Verfahren nach einem oder mehreren der vorhergehenden Ansprüche,
**dadurch gekennzeichnet,**
**dass** als organisches Lösungsmittel lineare oder verzweigte Alkohole mit Kettenlängen von 2 bis 8 Kohlenstoffatomen oder cyclische, aromatische oder heteroaromatische Kohlenwasserstoffe oder Derivate hiervon eingesetzt werden.

9. Verfahren nach einem oder mehreren der vorhergehenden Ansprüche,
**dadurch gekennzeichnet,**
**dass** die Alkyl- und/oder Alkylenreste des metallorganischen Titanoxid-Precursors bevorzugt eine Kettenlänge von 2 bis 5 Kohlenstoffatomen aufweisen.

10. Verfahren nach einem oder mehreren der vorhergehenden Ansprüche,
**dadurch gekennzeichnet,**
**dass** als Säure Salpetersäure, Salzsäure, Schwefelsäure, Phosphorsäure, eine organische Säure oder Gemische hiervon verwendet werden.

11. Verfahren nach einem oder mehreren der vorhergehenden Ansprüche,
**dadurch gekennzeichnet,**
**dass** die Metallionen-Konzentration in Schritt a) so gewählt wird, dass die aufgebrachte getrocknete und ggf. erhitzte Beschichtung eine Metallionenkonzentration von 1 - 20 Gew.-%, bevorzugt 5 - 15 Gew.%, noch bevorzugter 10 - 12 Gew.-% aufweist.

12. Verfahren nach einem oder mehreren der vorhergehenden Ansprüche,
**dadurch gekennzeichnet,**
**dass** das Aufbringen durch Tauchbeschichtung, Spin-Coaten Rakeln, Drucken oder Aufsprühen erfolgt.

13. Verfahren nach einem oder mehreren der vorhergehenden Ansprüche,
**dadurch gekennzeichnet,**
**dass** das Sol aus Schritt a) mit einer solchen Schichtdicke aufgebracht wird, dass die Schichtdicke einer Einfachbeschichtung nach Trocknen und ggf. Erhitzen 50-1000 nm, bevorzugt 50-200 nm, noch bevorzugter 130 - 170 nm, am bevorzugtesten ungefähr 150 nm beträgt.

14. Verfahren nach einem oder mehreren der vorhergehenden Ansprüche,
**dadurch gekennzeichnet,**
**dass** das Sol aus Schritt a) in Form eines Sols aufgebracht wird, wobei das Sol, in welchem die Metallsalze und/oder metallorganischen Verbindungen homogen verteilt und gelöst sind, bei oder nach dem Auftragen in ein Gel übergeht, in welchem die Metallionen homogen verteilt und gelöst vorliegen.

15. Verfahren nach einem oder mehreren der vorhergehenden Ansprüche,
**dadurch gekennzeichnet,**
**dass** die Schritte a) - c) von Anspruch 1 ein oder mehrmals wiederholt werden, um eine oder mehrere zusätzliche Titanoxid-Beschichtungen auf dem Implantat zu erzeugen, wobei die Beschichtungen wahlweise jeweils nach Schritt c) auf 100 bis 1000°C erhitzt werden.

16. Verfahren nach Anspruch 15,
**dadurch gekennzeichnet,**
**dass** die Metallionen-Konzentration jeweils in Schritt a) so variiert wird, dass die ursprüngliche Beschichtung und die ein oder mehreren zusätzlich aufgebrachten getrockneten und wahlweise erhitzten Beschichtungen unterschiedliche Metallionenkonzentrationen aufweisen.

17. Verfahren nach Anspruch 15 oder 16,
**dadurch gekennzeichnet,**
**dass** die Metallionen-Konzentration jeweils in Schritt a) so variiert wird, dass die Metallionenkonzentration in der ursprünglichen Schicht und in den ein oder mehreren zusätzlich aufgebrachten getrockneten und wahlweise erhitzten Beschichtungen von den innen am Implantat liegenden Beschichtungen hin zu den außen liegenden Beschichtungen abnimmt.

18. Verfahren nach einem oder mehreren der vorhergehenden Ansprüche,
**dadurch gekennzeichnet,**
**dass** das Trocknen der aufgebrachten Beschichtung in Schritt c) unter überkritischen Bedingungen erfolgt.

19. Verfahren nach einem oder mehreren der Ansprüche 15-18,
**dadurch gekennzeichnet,**
**dass** die einzelnen aufgebrachten Beschichtungen unterschiedliche Metallionen aufweisen.

20. Verfahren nach einem oder mehreren der Ansprüche 15-19,
**dadurch gekennzeichnet,**
**dass** die antibakteriellen bzw. antimikrobiellen Metallionen Kupferionen und/oder Silberionen sind.

21. Implantat mit einer Titanoxid-Beschichtung, herstellbar nach dem Verfahren gemäß einem oder mehreren der vorhergehenden Ansprüche.

22. Implantat nach Anspruch 21,
**dadurch gekennzeichnet,**
**dass** die in der Beschichtung enthaltenen Metallionen unter physiologischen Bedingungen aus der Beschichtung in das umgebende Medium herauslösbar sind.

23. Implantat nach Anspruch 21 oder 22,
**dadurch gekennzeichnet,**
**dass** die Schichtdicke einer Titanoxid-Einfachbeschichtung jeweils 50-1000 nm, bevorzugt 50-200 nm, noch bevorzugter 130 - 170 nm, am bevorzugtesten ungefähr 150 nm beträgt.

24. Implantat nach einem oder mehreren der Ansprüche 21 - 23,
**dadurch gekennzeichnet,**
**dass** die Metallionen in einer Titanoxid-Beschichtung jeweils homogen verteilt sind.

25. Implantat nach einem oder mehreren der Ansprüche 21 - 24,
**dadurch gekennzeichnet,**
**dass** die Metallionen in einer solchen Konzentration in der Titanoxid-Beschichtung vorliegen, dass die Beschichtung zunächst antibakteriell wirkt und nach einer einstellbaren Zeit biokompatibel ist.

26. Implantat nach einem oder mehreren der Ansprüche 21 - 25,
**dadurch gekennzeichnet,**
**dass** die Metallionenkonzentration in einer Titanoxid-Beschichtung 1 - 20 Gew.-%, bevorzugt 5 - 15 Gew.%, noch bevorzugter 10 - 12 Gew.-% beträgt.

27. Implantat nach einem oder mehreren der Ansprüche 21 - 26,
**dadurch gekennzeichnet,**
**dass** die in der Titanoxid-Beschichtung enthaltenen Metallionen Kupferionen und/oder Silberionen sind.

## Claims

1. A method for the preparation of a titanium oxide coating on an implant comprising the steps of:
a) adding a sol containing an organic solvent and an organometallic titanium oxide precursor and optionally water and/or an acid with metal salts and/or with organometallic compounds to disperse metal ions homogeneously in the sol wherein the metal ions exert an anti-microbial or anti-bacterial effect, respectively, under physiological conditions and comprise mono or tetravalent metal ions selected from copper, zinc, silver and mercury ions, and wherein the organometallic titanium oxide precursor is fourfold coordinated titanium having linear or branched alkyl and/or alkylene radicals bound by oxygen bridges wherein the alkyl and/or alkylene radicals may have O and/or N atoms either substituted or within the chain;
b) applying the sol prepared in a) onto an implant;
c) drying the coating thus applied.

2. The method according to claim 1
**characterized in that**
after said step c) heating is conducted to 100 to 1000°C.

3. The method according to step 1 or 2
**characterized in that**
the implant is a metal, metal alloy, a glass, a ceramic, a plastic, a composite material, or a bone implant.

4. The method according to one or more of the preceding claims
**characterized in that**
said implant is a catheter, an osteosynthesis plate, an endoprosthesis, an external fixateur, an internal fixateur, a nail, a screw, and/or a wire, a heart valve, an artificial blood vessel, or a shunt, an implant for facial/plastic surgery, a middle ear implant, or a dental implant.

5. The method according to one or more of the preceding claims
**characterized in that**
said metal in the case of a metallic implant is titanium, steel, iron and/or an alloy containing steel, iron, titanium and/or CoCr.

6. The method according to one or more of the preceding claims
**characterized in that** the metal alloy is a titanium alloy, preferably TiAl6V4 or TiAl6Nb7, a CoCr alloy or an osteosynthesis steel.

7. The method according to one or more of the preceding claims
**characterized in that**
said plastic is polyethylene, polypropylene, polytetrafluoroethylene, polyethylene terephthalate, polyamides, polyurethanes, polysiloxanes, polysiloxane elastomers, polyetherether ketone, and/or polysulfone.

8. The method according to one or more of the preceding claims
**characterized in that**
as the organic solvent linear or branched alcohols with chain lengths of 2 to 8 carbon atoms or cyclic, aromatic or heteroaromatic hydrocarbons or derivatives thereof are used.

9. The method according to one or more of the preceding claims
**characterized in that**
the alkyl and/or alkylene radicals of the organometallic titanium oxide precursor preferably have a chain length of 2 to 5 carbon atoms.

10. The method according to one or more of the preceding claims
**characterized in that**
as the acid nitric acid, hydrochloric acid, sulphuric acid, phosphoric acid, an organic acid or mixtures thereof are used.

11. The method according to one or more of the preceding claims
**characterized in that**
the metal ion concentration in step a) is selected to give a metal ion concentration of 1 - 20 % by weight, preferably 5 - 15 % by weight, still more preferred of 10 - 12 % by weight in the applied, dried and optionally heated coating.

12. The method according to one or more of the preceding claims
**characterized in that**
said application is carried out by dip coating, spin coating, blade coating, printing or spraying.

13. The method according to one or more of the preceding claims
**characterized in that**
the sol of step a) is applied in a coating thickness that the coating thickness of a single coating after drying and optionally heating is 50 - 1000 nm, preferably 50 - 200 nm, more preferably 130 - 170 nm, most preferably about 150 nm.

14. The method according to one or more of the preceding claims
**characterized in that**
the sol of step a) is applied in the form of a sol wherein said sol in which the metal salts and/or organometallic compounds are homogeneously dispersed and dissolved transforms into a gel during or after the application wherein the metal ions are homogeneously dispersed and dissolved.

15. The method according to one or more of the preceding claims
**characterized in that**
the steps a) - c) of claim 1 are repeated once or several times to generate one or more additional titanium oxide coatings on the implant wherein each of the coatings can optionally be heated after step c) to 100 to 1000 °C.

16. The method according to claim 15
**characterized in that**
the metal ion concentration is varied in step a) to achieve different concentrations of metal ions in the original coating and the one or more additionally applied, dried and optionally heated coatings.

17. The method according to claim 15 or 16
**characterized in that**
the metal ion concentration is varied in step a) to achieve concentrations of metal ions in the original coating and in the one or more additionally applied, dried and optionally heated coatings decrease from the internal coatings close to the implant to the external coatings.

18. The method according to one or more of the preceding claims
**characterized in that**
drying of the coating applied in step c) is performed under supercritical conditions.

19. The method according to one or more of the claims 15-18
**characterized in that**
the individually applied coatings contain different metal ions.

20. The method according to one or more of claims 15-19
**characterized in that**
the antibacterial or anti-microbial metal ions, respectively, are copper ions and/or silver ions.

21. An implant having a titanium oxide coating which can be prepared according to one or more of the preceding claims.

22. The implant according to claim 21
**characterized in that**
the metal ions contained in the coating can be dissolved out of the coating into the surrounding medium under physiological conditions.

23. The implant according to claim 21 or 22
**characterized in that**
the layer thickness of each single titanium oxide coating is 50 - 1000 nm, preferably 50 - 200 nm, more preferred 130 - 170 nm, most preferably about 150 nm.

24. The implant according to one or more of the claims 21 - 23
**characterized in that**
the metal ions are homogeneously dispersed in each titanium oxide coating.

25. The implant according to one or more of the claims 21 - 24
**characterized in that**
the metal ions are contained in the titanium oxide coating in a concentration that the coating initially has an antibacterial effect and that it is biocompatible after an adjustable time.

26. The implant according to one or more of the claims 21 - 25
**characterized in that**
the metal ion concentration in a titanium oxide coating is 1 - 20 % by weight, preferably 5 - 15 % by weight, still more preferred of 10 - 12 % by weight.

27. The implant according to one or more of the claims 21 - 26
**characterized in that**
the metal ions contained in the titanium oxide coating are copper ions and/or silver ions.

## Revendications

1. Procédé de fabrication d'un revêtement d'oxyde de titane sur un implant, ayant les étapes suivantes :
a) on mélange un sol, contenant un solvant organique et un précurseur d'oxyde de titane organométallique et, au choix, de l'eau et/ou un acide, à des sels métalliques et/ou à des composés organométalliques, pour répartir de manière homogène des ions métalliques dans le sol, lesdits ions métalliques développant une action antimicrobienne ou antibactérienne dans des conditions physiologiques et présentant des ions métalliques mono- à tétravalents choisis dans le groupe constitué des ions de cuivre, de zinc, d'argent et de mercure, et le précurseur d'oxyde de titane organométallique étant un titane présentant 4 liaisons de coordination avec des radicaux alkyle et/ou alkylène linéaires ou ramifiés, pontés par le biais d'atomes d'oxygène, qui peuvent présenter dans la chaîne ou sous forme substituée, des atomes d'oxygène et/ou d'azote,
b) on applique le sol fabriqué à l'étape a) sur un implant,
c) on séche le revêtement appliqué.

2. Procédé selon la revendication 1,
**caractérisé en ce que**,
on effectue après l'étape c) un chauffage à une température de 100 à 1000 °C.

3. Procédé selon la revendication 1 ou 2,
**caractérisé en ce que**,
l'implant est un métal, un alliage de métaux, un verre, une céramique, un matériau synthétique, un matériau composite ou un implant osseux.

4. Procédé selon l'une quelconque ou plusieurs des revendications précédentes,
**caractérisé en ce que**,
l'implant est un cathéter, une plaque d'ostéosynthèse, une endoprothèse, un fixateur externe, un fixateur interne, un clou, une vis ou un fil, une valvule cardiaque, un vaisseau sanguin artificiel ou une dérivation (shunt), un implant de chirurgie du visage/chirurgie plastique, un implant d'oreille moyenne ou un implant dentaire.

5. Procédé selon l'une quelconque ou plusieurs des revendications précédentes,
**caractérisé en ce que**,
le métal est, dans le cas d'un implant métallique, du titane, de l'acier, du fer et/ou un alliage à base d'acier, de fer, de titane et/ou de CoCr.

6. Procédé selon l'une quelconque ou plusieurs des revendications précédentes,
**caractérisé en ce que**,
l'alliage métallique est un alliage de titane, de préférence du TiAl6V4 ou du TiAl6Nb7 ; un alliage de CoCr ; ou un acier d'ostéosynthèse.

7. Procédé selon l'une quelconque ou plusieurs des revendications précédentes,
**caractérisé en ce que**,
le matériau synthétique est représenté par le polyéthylène, le polypropylène, le poly(tétrafluorure d'éthylène), le poly(téréphtalate d'éthylène), des polyamides, des polyuréthannes, des polysiloxanes, des élastomères de polysiloxane, une polyétheréthercétone et/ou une polysulfone.

8. Procédé selon l'une quelconque ou plusieurs des revendications précédentes,
**caractérisé en ce que**,
on utilise comme solvant organique, des alcools linéaires ou ramifiés ayant des longueurs de chaîne de 2 à 8 atomes de carbone, ou des hydrocarbures cycliques, aromatiques ou hétéroaromatiques ou des dérivés de ceux-ci.

9. Procédé selon l'une quelconque ou plusieurs des revendications précédentes,
**caractérisé en ce que**,
les radicaux alkyle et/ou alkylène du précurseur d'oxyde de titane organométallique présentent, de préférence, une longueur de chaîne de 2 à 5 atomes de carbone.

10. Procédé selon l'une quelconque ou plusieurs des revendications précédentes,
**caractérisé en ce que**,
on utilise comme acide, l'acide nitrique, l'acide chlorhydrique, l'acide sulfurique, de l'acide phosphorique, un acide organique ou des mélanges de ceux-ci.

11. Procédé selon l'une quelconque ou plusieurs des revendications précédentes,
**caractérisé en ce que**,
on choisit la concentration d'ions métalliques à l'étape a) de manière que le revêtement appliqué séché, et éventuellement chauffé, présente une concentration en ions métalliques de 1 à 20 % en poids, de préférence, de 5 à 15 % en poids, mieux encore, de 10 à 12 % en poids.

12. Procédé selon l'une quelconque ou plusieurs des revendications précédentes,
**caractérisé en ce que**,
l'application est réalisée à l'aide d'un procédé de revêtement par trempage, par centrifugation, par raclement, par impression ou par pulvérisation.

13. Procédé selon l'une quelconque ou plusieurs des revendications précédentes,
**caractérisé en ce que**,
on applique le sol obtenu à l'étape a) selon une épaisseur de couche qui corresponde, après séchage et, éventuellement, après chauffage, à une épaisseur de couche d'un revêtement unique de 50 à 1000 nm, de préférence, de 50 à 200 nm, mieux encore, de 130 à 170 nm, bien mieux encore, d'environ 150 nm.

14. Procédé selon l'une quelconque ou plusieurs des revendications précédentes,
**caractérisé en ce que**,
on applique le sol obtenu à l'étape a) sous la forme d'un sol, le sol en question, dans lequel les ions métalliques et/ou les composés organométalliques sont répartis et dissous de manière homogène, se transformant en un gel pendant ou après l'application, dans lequel les ions métalliques sont répartis et dissous de manière homogène.

15. Procédé selon l'une quelconque ou plusieurs des revendications précédentes,
**caractérisé en ce que**,
les étapes a) à c) de la revendication 1 sont réitérées une ou plusieurs fois, afin de produire un ou plusieurs revêtements d'oxyde de titane supplémentaires sur l'implant, les revêtements étant éventuellement chauffés, respectivement, après l'étape c) à une température de 100 à 1000 °C.

16. Procédé selon la revendication 15,
**caractérisé en ce que**,
on fait varier la concentration d'ions métalliques, respectivement, à l'étape a), de sorte que le revêtement initial et le ou les multiples revêtements supplémentaires appliqués, séchés et, éventuellement, chauffés, présentent des concentrations d'ions métalliques différentes.

17. Procédé selon la revendication 15 ou 16,
**caractérisé en ce que**,
on fait varier la concentration d'ions métalliques, respectivement, à l'étape a), de sorte que la concentration d'ions métalliques dans la couche initiale et dans le ou les multiples revêtements supplémentaires appliqués, séchés et, éventuellement, chauffés, soit décroissante des revêtements internes déposés sur l'implant aux revêtements situés à l'extérieur.

18. Procédé selon l'une quelconque ou plusieurs des revendications précédentes,
**caractérisé en ce que**,
le séchage du revêtement appliqué à l'étape c) est effectué dans des conditions supercritiques.

19. Procédé selon l'une quelconque ou plusieurs des revendications 15 à 18,
**caractérisé en ce que**,
les revêtements individuels appliqués présentent des ions métalliques différents.

20. Procédé selon l'une quelconque ou plusieurs des revendications 15 à 19,
**caractérisé en ce que**,
les ions métalliques antibactériens ou antimicrobiens sont des ions de cuivre et/ou des ions d'argent.

21. Implant doté d'un revêtement d'oxyde de titane, que l'on peut fabriquer selon le procédé répondant à l'une ou à plusieurs des revendications précédentes.

22. Implant selon la revendication 21,
**caractérisé en ce que**,
les ions métalliques contenus dans le revêtement peuvent être évacués du revêtement vers le milieu environnant dans des conditions physiologiques.

23. Implant selon la revendication 21 ou 22,
**caractérisé en ce que**,
l'épaisseur de couche d'un revêtement unique d'oxyde de titane est, respectivement, de 50 à 1000 nm, de préférence de 50 à 200 nm, plus préférentiellement de 130 à 170 nm, et encore plus préférentiellement d'environ 150 nm.

24. Implant selon l'une ou plusieurs des revendications 21 à 23,
**caractérisé en ce que**,
les ions métalliques sont répartis de manière homogène dans un revêtement d'oxyde de titane.

25. Implant selon l'une ou plusieurs des revendications 21 à 24,
**caractérisé en ce que**,
les ions métalliques sont présents en concentration telle que le revêtement exerce d'abord une action antibactérienne et s'avère biocompatible à l'issue d'une période de temps que l'on peut régler.

26. Implant selon l'une ou plusieurs des revendications 21 à 25,
**caractérisé en ce que**,
la concentration d'ions métalliques dans un revêtement d'oxyde de titane est de 1 à 20 % en poids, de préférence, de 5 à 15 % en poids, mieux encore, de 10 à 12 % en poids.

27. Implant selon l'une ou plusieurs des revendications 21 à 26,
**caractérisé en ce que**,
les ions métalliques contenus dans le revêtement d'oxyde de titane sont des ions de cuivre et/ou des ions d'argent.
